# EUROPEAN PATENT SPECIFICATION

(11) **EP 0 637 632 B1**
(45) Date of publication and mention of the grant of the patent: **12.07.2000**
(21) Application number: 94112271.5
(22) Date of filing: 05.08.1994
(51) Int. Cl.: C12Q 1/56

(54) **Method of measuring activity of test substance**
Verfahren zur Prüfung der Wirksamkeit einer Testsubstanz
Méthode pour la mesure de l'activité d'une substance d'essai

(30) Priority: 06.08.1993 JP 21517893
(43) Date of publication of application: 08.02.1995
(73) Proprietor: NIPPON ZOKI PHARMACEUTICAL CO. LTD., Osaka (JP)
(72) Inventor: Nishikawa, Katsumi, c/o Ins. of Bio-Active Science, Yashiro-cho, Katoh-gun, Hyogo (JP); Kawakubo, Hitoshi, c/o Ins. of Bio-Active Science, Yashiro-cho, Katoh-gun, Hyogo (JP)
(74) Representative: Hansen, Bernd, Dr. Dipl.-Chem.

(56) References cited:
- EP-A- 0 078 764
- EP-A- 0 190 766
- EP-A- 0 259 857
- WO-A-91/17258
- US-A- 4 882 272
- H. U. BERGMEYER (EDITOR-IN-CHIEF): "Methods of Enzymatic Analysis, Vol. V." 1984 , VERLAG CHEMIE GMBH. , WEINHEIM, DE XP002046938 014041 * page 316 - page 351 * * page 394 - page 399 *

## Description

### Acknowledgment of relevant state of the art:

(EP-A-0 259 857) discloses a method for assaying physiologically active substances based on the measurement of the extent of conversion of plasma prekallikrein to kallikrein upon addition of an activator of the blood coagulation Factor XII to an *animal plasma sample* in the presence of a drug to be tested.

(EP-A-0 078 764) discloses a method for quantitative determination of Factor XII in human plasma based on measurements of the enzymatic activity of activated Factor XII (Factor XIIa) upon addition of an activator to a *human plasma sample* in the presence of a suitable substrate.

(US-A-4,882,272) discloses an assay to determine the amount of high molecular weight kininogen (HMWK) in a human plasma sample using activated Factor XI (Factor XIa) as substrate. The activation of Factor XI proceeds upon addition of a known amount of exogenous Factor XIIa to a sample containing *inhibited endogeneous plasma prekallikrein and kallikrein*. Concentrations of exogenous Factor XIIa and Factor XI in the sample are selected such as that the concentration of functional HMWK in the sample is rate-limiting in the HMWK-mediated activation of Factor XI by Factor XIIa.

### Detailed Description of the Invention:

The present invention relates to a method of measuring the activity of a tested substance to the production of a physiologically active substance produced in a reconstituted plasma kallikrein-kinin system. More particularly, it relates to a method of measuring a physiologically active substance produced in said reaction system - such as a blood coagulation factor XII in an active form, plasma kallikrein,or bradykinin - utilizing a reconstituted system comprising the components of plasma kallikrein-kinin system.

Kallikrein is a protease which is widely distributed in plasma and tissues of various animals including human beings and an enzymatic system called a kallikrein-kinin system has been known. The kallikrein-kinin system acts in vivo having a close relationship with various other enzymatic reaction systems such as a renin-angiotensin system, a blood clotting system, a fibrinolysis system, a complement system as well as catecholamine and arachidonic acid cascades mainly related to prostaglandins, leukotrienes and thromboxanes. Accordingly, the kallikrein-kinin system is closely related to blood pressure regulating action, action through the blood clotting-fibrinolysis-complement system or bioregulation, and the improving action for peripheral circulation by various physiologically active substances produced by an arachidonic acid cascade and plays an important role on the regulation of functions in vivo.

Kinins such as bradykinin which are produced in the kallikrein-kinin system exhibit various physiological activities such as decreasing the blood pressure due to dilation of peripheral blood vessels, promotion in permeability of blood vessels,contraction or relaxation of smooth muscle, induction of pain , induction of inflammation, migration of leucocytes, liberation of catecholamine from the adrenal cortex, etc. and are also known as mediators in acute inflammations including allergic reactions whereby their existence in vivo has a deep significance.

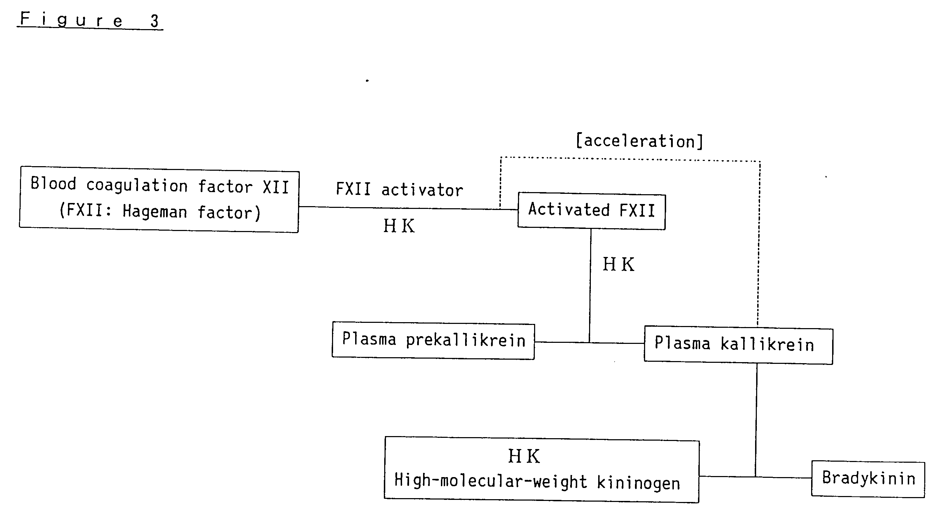

With respect to this plasma kallikrein-kinin system, it has been believed that a blood coagulation factor XII (a Hageman factor: hereinafter abbreviated as an FXII) is activated in vivo by injury and invaded stimulation to tissues whereby a series of enzymatic reaction systems results . Thus, as shown in Fig. 3, the activated blood coagulation factor XII in (hereinafter, abbreviated as an FXIIa) acts on the plasma prekallikrein which exists in the same plasma to convert it to a plasma kallikrein which is an enzyme of an activated form and then the plasma kallikrein acts on a high-molecular-weight kininogen (hereinafter abbreviated as an HK) to liberate bradykinin.

Kinins such as bradykinin which are liberated by the enzymatic reaction of the kallikrein-kinin system exhibit various physiological actions as mentioned already. Accordingly, there is a possibility that the substances which inhibit the action of bradykinin or the substances which inhibit the production of bradykinin interfering the reactions in the plasma kallikrein-kinin system can be used as antiinflammatory, analgesic and antiallergic agents. Further, FXIIa is an important factor in the initiating stage of the intrinsic blood clotting system and the fibrolysis system so that substances affecting the production of FXIIa are expected to be useful as drugs in the areas of blood clotting and fibrinolysis. Therefore, establishment of a method for measuring the above-mentioned substances,which inhibit or promote the reactions in the plasma kallikrein-kinin system,in a simple, easy, quick and precise manner would be a very imporant means for determining the action which would help in the regulation of the above-mentioned bioregulation and also for developing such drugs.

As mentioned already, when screenings or the like of drugs using the plasma kallikrein-kinin system are carried out in vitro, an activation of FXII by an invaded stimultion to tissues and injury such as an intravital reaction cannot be conducted and, therefore, a method in which a substance activating the FXII is added to an isolated plasma to carry out a reaction which induces a plasma kallikrein-kinin system reaction in vitro has been adopted.

However, in the plasma of animals, various components besides the above-mentioned components are contained. Thus, components which cause affects (such as an inhibition or a promotion) on the plasma kallikrein-kinin reaction system and other unknown factors are contained therein. Accordingly, when the activity of the tested substance to the production of the physiologically active substances is measured utilizing the above-mentioned reaction system ,with the object of screening or the like of drugs using the plasma kallikrein-kinin system, animal plasma per se has been used whereby the reaction system is complicated and the effects of various factors containing unknown components which may affect the plasma kallikrein-kinin reaction system has had to be always taken into consideration. Consequently, very detailed techniques are needed for controlling the reaction system and it is difficult to carry out in a simple manner.

The present invention is to solve the above-mentioned problems in the prior art and its object is to offer a method for measuring the physiological action of the tested substance in an easy, simple, prompt and precise manner wherein the plasma kallikrein-kinin system is reconstituted by taking out various components from the sample before use and said reaction is conducted in vitro.

Thus, the measuring method in accordance with the present invention is characterized in that a series of enzymatic reactions wherein the activation of the blood coagulation factor XII is an initiating reaction is started in the presence of a tested substance in the reconstituted plasma kallikrein-kinin system, then the reaction is stopped and the physiologically active substance produced in said reaction is qunatitatively determined.

With respect to the constituting components in the reconstituted plasma kallikrein-kinin system in the measuring method of the present invention, FXII and plasma prekallikrein can be used when the physiologically active substance to be determined is FXIIa or plasma kallikrein, and a preferred reaction system may also be constructed by adding an HK thereto. In the case of quantitative determination of bradykinin, it is preferred to utilize a system which is reconstituted using FXII, plasma prekallikrein and HK. With regard to each of those constituting components, that which is substantially purified may be used and that which is separated/purified from plasma or manufactured by a means of gene technology may be utilized.

With respect to the activating agent for FXII for inducing the activating reaction of the plasma kallikrein-kinin system, any substance may be used so far as it activates the FXII. Thus, glass, kaolin, celite, collagen, homocystine, sodium urate, cell components such as membranes of platelets and other cells, fibronectin, elaidic acid, quercetin, rutin, sulfated glycolipids, proteoglycan, mucopolysaccharides, sodium stearate, dextran sulfate, amylose sulfate, carrageenin and proteases which activate FXII by a restricted decomposition may be used either solely or jointly. Concentration of those FXII activators may be suitably selected.

In the mixing reaction carried out by adding an FXII activator to a solution comprising a substantially purified FXII, plasma prekallikrein and HK, the reaction temperature may be suitably adjusted so as to make the reaction easily controllable. It is preferred that the mixing reaction is carried out at the pH where the plasma kallikrein-kinin system reaction smoothly proceeds such as from 7.0 to 9.0. In order to adjust to a suitable reaction condition, salts such as sodium chloride, metal ions such as zinc ion and other additives and auxiliary agents which are commonly used in said art may be added to the reaction system.

The time for the mixing reaction may be suitably adjusted depending upon the concentrations of the above-mentioined FXII, FXII activators, plasma prekallikrein, HK and the tested substance as well as upon the pH of the reaction solution. However, when the produced amount of the physiologically active substance to be quantitatively determined is saturated, it is not possible to correctly evaluate the action of the tested substance and, therefore, the time is to be preferably adjusted within such an extent that until the produced amount of the physiologically active substance to be determined is saturated or, in other words, within a time that a clear positive relationship between the reaction time and the produced physiologically active susbtance (e.g. a linear proportional relationship which is preferred for the evaluation) is constituted.

The method for stopping the production of the physiologically active substance in the above-mentioned mixing reaction may be suitably selected depending upon the physiologically active substance to be determined. Thus, in the case of a quantitative determination of FXIIa for example, it is preferred to use an FXII activation inhibitors (e.g. polybrene) and inhibitors which specifically inhibit the plasma kallikrein (preferably those which do not substantially affect FXIIa such as SBTI [soy bean trypsin inhibitor; a trypsine inhibitor prepared from soy bean]) whereby the produced FXII can be quantitatively determined using an enzymatic activity of FXIIa as a target applying a substrate to FXIIa.

In the quantitative determination of a plasma kallikrein, it is preferred to use substances which stop the production of plasma kallikrein (e.g. inhibitors which specifically inhibit FXIIa, preferably, the inhibitors which substantially do not affect the plasma kallikrein such as LBTI [lima bean trypsin inhibitor: a trypsin inhibitor prepared from lime bean] and CHFI [corn Hageman fragment inhibitor: a Hageman fragment inhibitor prepared from corn]) whereby the produced plasma kallikrein can be determined using the enzymatic activity of the plasma kallikrein as a target, utilizing a substrate to the plasma kallikrein.

When bradykinin is quantitatively determined as a produecd physiologicaly active substance, it is possible to use a substance which stops the production of bradykinin such as the above-mentioned inhibitors to FXIIa and plasma kallikrein,having an action of liberating bradykinin by a restricted decomposition of HK, as well as organic solvents such as acetone and ethanol or acids such as hydrochloric acid and perchloric acid.

The concentration of those substances which are used for stopping the production of the physiologically active substances may be suitably selected so that the quantitative determination of each of the physiologically active substances is not substantially affected.

Quantitative determination of the produced physiologically active substance may be carried out by conventional measuring methods. With respect to a method of measuring the produced amount of FXIIa, a method wherein a substrate to FXIIa is used utilizing the enzymatic activity of FXIIa may be applied for example. Methods of using a natural substrate such as plasma prekallikrein, FXII or plasmin, a coloring synthetic substrate such as D-Pro-Phe-Arg-pNA or D-Leu-Gly-Arg-pNA or a fluorescent synthetic substrate such as Boc-Glu(OBz)-Gly-Arg-MCA or Boc-Gln-Gly-Arg-MCA are simple and convenient and are well used in many cases.

In the quantitative determination of the produced plasma kallikrein, methods in which a natural substrate such as FXII or HK, a coloring synthetic substrate such as D-Pro-Phe-Arg-pNA or Bz-Pro-Phe-Arg-pNA or a fluorescent synthetic substrate such as Z-Phe-Arg-MCA may be used as well.

Besides the above-mentioned measuring methods using substrates, immunological measuring methods such as a radioimmunoassay (RIA) or an enzyme immunoassay (EIA), quantitative determination using chromatogrphy, etc. may be used

With respect to the method for quantitative determination of the produced bradykinin, commonly-used means such as bioassay, RIA, EIA, etc. may be used. Any method may be chosen depending upon the conditions such as the numbers of the tested substances, devices in the measuring facilities, preciseness requested for the measurement, etc.

### (Examples)

The present invention will be further illustrated by way of the following examples.

### Example 1

Dextran sulfate was added to make the final concentration 5 micrograms/ml to a solution comprising 6nM of FXII, 35nM of plasma kallikrein, 10nM of HK, certain amount of the tested substance and 25mM of a Tris hydrochloride buffer (pH: 8) containing Triton X-100, BSA and 100mM of NaCl. The mixture was incubated in ice water and LBTI was added to the reaction solution to make the final concentration 20 mg/ml. The reaction solution was incubated at 30°C for 30 minutes in a Tris hydrochloride buffer (pH: 8) together with 1mM of D-Pro-Phe-Arg-pNA (a synthetic substrate), the reaction was stopped by adding citric acid thereto, the mixture was centrifuged at 3,000 rpm for ten minutes and the absorbance of the resulting supernatant liquid at 405 nm was measured which corresponded to the plasma kallikrein production.

### Example 2

The same operations as in Example 1 were carried out with the exception that 0.6nM of, FXII was used and then the absorbance at 405 nm was measured.

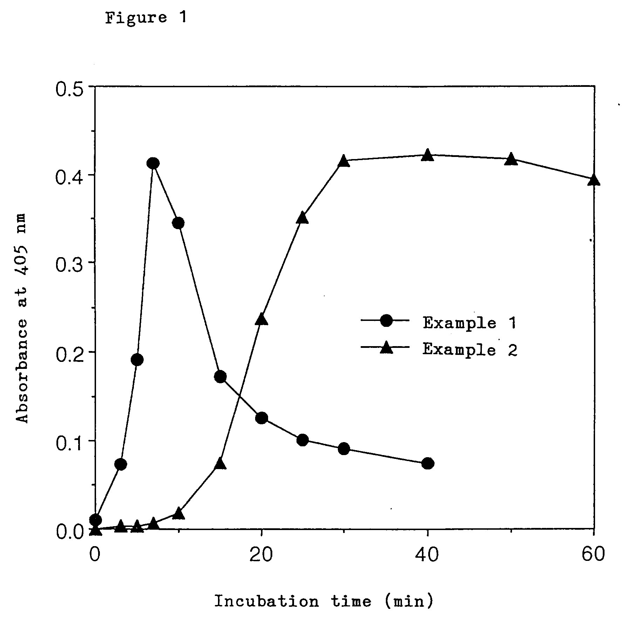

Fig. 1 shows the changes in the absorbances when the incubating time was changed in Example 1 (●) and in Example 2 (▲). In Fig. 1, the abscissa shows the incubating time (minutes) while the ordinate shows the absorbances at 405 nm. In the case of Example 1, kallikrein was produced in an almost linear increase with lapse of time until the incubating time became about seven minutes and its production arrived at the saturated point after about seven minutes. Accordingly, it is preferred that the incubating time is to be set between 0 and 7 minutes. In the case of Example 2, the time required for saturation of the kallikrein production can be elongated to 30 minutes. In addition, the amount of the plasma prekallikrein In Example 2 was the same as that in Example 1 and, therefore, the intensity of the absorbance which is finally measurable was 0.4 and was the same as that in Example 1.

When animal plasma is used in the conventional plasma kallikrein-kinin system, the animal plasma is ten times diluted to decrease the amount of the FXII to one-tenth and, at the same time, the amount of the plasma prekallikrein to one-tenth. Therefore, the intensity of the absorbance to be measured in the end is as low as one-tenth the undiluted value, so that the measurement is almost impossible.

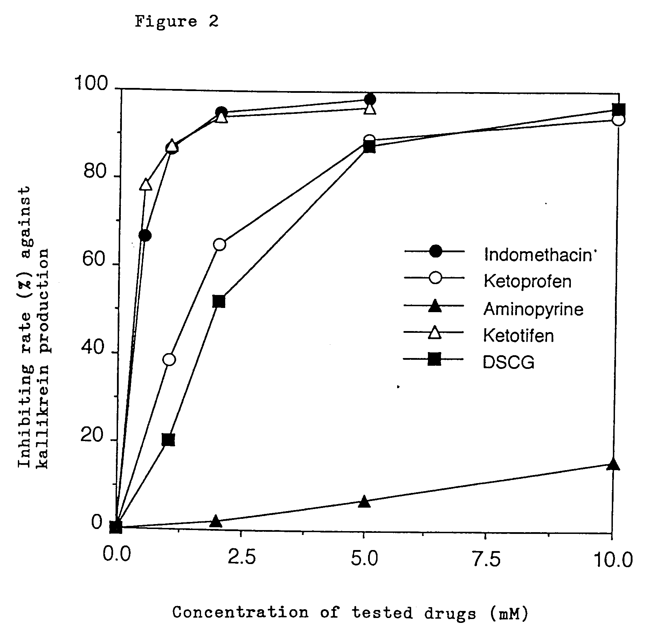

Fig. 2 shows the result of the measurement of the activity for inhibiting the plasma kallikrein production of five drugs (indomethacin, ketoprofen, aminopyrine, ketotifen and DSCG), which are used as analgesic or antiallergic drugs, utilizing the method of measuring the activity according to the present invention. In the drawing, the abscissa is the concentrations (mM) of the test drugs while the ordinate is the inhibiting rate (%) against the production of kallikrein.

The method of measuring the activity according to the present invention utilizing the reconstituted plasma kallikrein-kinin system is a reaction system in which the contaminating other factors are substantially removed. Therefore, it is not necessary to regulate the reaction by taking into account the effects of the factors which may affect the enzymatic reaction system of the plasma kallikrein-kinin system and unknown components therein whereby the real activity (a promoting or inhibiting ability) of the tested substance to the production of the physiologically active substance produced in the plasma kallikrein-kinin system can be measured in a easy, convenient, prompt and precise manner.

In the measuring method of the present invention, each substantially purified component is used in place of the animal plasma which is used in the conventional plasma kallikrein-kinin system and, therefore, it has various advantages given below as comapred with the cases where the animal plasma is used.
1. The amount of each of the constituting components can be freely adjusted and, accordingly, reaction time, reaction temperture, absorbance, etc. in the reaction system can be suitably chosen depending upon the number of the tested substances and the required precision;
2. The influence of the endogeneous inhibitors such α₂ macroglobulin and C1-inhibitor and kininase being present in the animal plasma can be substantially neglected and, in addition, it is not necessary to take the factors which may affect the reaction and unknown substances in the animal plasma into consideration. Consequently, the real activity of the tested substance to the production of the physiologically active substance produced in the plasma kallikrein-kinin system can be measured precisely. To be more specific, there is an advantage that, for example, there is no need to use a kininase inhibitor in the measurement of the action of the tested substance to the bradykinin production; and
3. The scattering in the reactions due to the lack of uniformity in the components of the animal plasma can be prevented whereby an increase in the precision of the measurement results.

In the quantitative determination of the produced physiologically active susbtances in the measuring method of the present invention, various methods such as a method of measuring the inhibiting ability against the production of FXIIa, a method of measuring the inhibiting ability against the plasma kallikrein production and a method of measuring the inhibiting ability against the bradykinin production can be suitably selected. Accordingly, the degree of freedom in the measurement is large and the applicable range is broad as well.

Furthermore, in the method for measuring the activity of the present invention, a reconstituted system is used wherein substantially purified components are used and the other contaminating factors are removed and it is now possible to screen drugs having an activity to the production (a promoting or an inhibiting ability for the production) of various physiologically active substances in the plasma kallikrein-kinin system from various points of view whereby the screening of the drugs based upon the clear and specific action mechanism is possible. Accordingly, the present invention achieves a significant effect on the development of new drugs for various diseases.

### Brief Explanation of the Drawings:

Fig. 1 shows the changes in the absorbances for various incubating times in Examples 1 and 2 of the present invention.
Fig. 2 shows the result of the measurement of the inhibiting activity against the plasma kallikrein production of several tested substances using the method of measuring the activity of the present invention.
Fig. 3 is a scheme illustrating the mechanism of the enzymatic reaction of the plasma kallikrein-kinin.

## Claims

1. A method of measuring the activity of a test substance, **characterized** by the following steps:
i) initiating a series of enzymatic reactions in a reconstituted plasma kallikrein-kinin system, in the presence of the test substance, wherein the activation of blood coagulation factor XII is the initial reaction,
ii) stopping the reaction, and
iii) quantitatively determining the physiologically active substance produced in the above reaction.

2. A method according to claim 1 in which the production of the physiologically active substance is stopped before this production becomes saturated.

3. A method according to claims 1 or 2 in which the physiologically active substance is blood coagulation factor XII in an active form.

4. A method according to claims 1 or 2 in which the physiologically active substance is plasma kallikrein.

5. A method according to claims 1 or 2 in which the physiologically active substance is bradykinin.

6. A method according to any one of claims 1 to 4 in which the system is reconstituted by the addition of blood coagulation factor XII and plasma prekallikrein.

7. A method according to any of claims 1 to 5 in which the system is reconstituted by the addition of blood coagulation factor XII, plasma prekallikrein and a high molecular weight kininogen.

8. A method according to any one of claims 1 to 4, 6 and 7, in which the physiologically active substance produced is determined by the addition of a substrate.

9. A method according to claim 8 in which the substrate is a synthetic substrate.

10. The use of a reconstituted plasma kallikrein-kinin system comprising:
i) blood coagulation factor XII
ii) plasma prekallikrein,
iii) optionally comprising a high molecular weight kininogen, and
iv) containing no other inhibitors or promotors of the plasma kallikrein-kinin reaction system.
for the measurement of the activity of a test substance.

11. The use according to claim 10 wherein the test substance is a drug.

## Patentansprüche

1. Verfahren zur Messung der Aktivität einer Testsubstanz, gekennzeichnet durch die folgenden Schritte:
i) Initiieren einer Serie von enzymatischen Reaktionen in einem in aufbereitetem Plasma vorliegenden Kallikrein-Kinin-System in Gegenwart der Testsubstanz, worin die Aktivierung des Blutgerinnungsfaktors XII die Initialreaktion darstellt,
ii) Abbruch der Reaktion, und
iii) quantitative Bestimmung der physiologisch aktiven Substanz, die in der obigen Reaktion hergestellt wird.

2. Verfahren gemäß Anspruch 1, in dem die Herstellung der physiologisch aktiven Substanz abgebrochen wird, bevor diese Herstellung den Gleichgewichtszustand erreicht.

3. Verfahren gemäß Ansprüchen 1 oder 2, in dem die physiologisch aktive Substanz der Blutgerinnungsfaktor XII in einer aktiven Form ist.

4. Verfahren gemäß Ansprüchen 1 oder 2, in dem die physiologisch aktive Substanz Plasmakallikrein ist.

5. Verfahren gemäß Ansprüchen 1 oder 2, in dem die physiologisch aktive Substanz Bradykinin ist.

6. Verfahren gemäß einem oder mehreren der Ansprüche 1 bis 4, in dem das System durch Zugabe von Blutgerinnungsfaktor XII und Plasmapräkallikrein aufbereitet wird.

7. Verfahren gemäß einem oder mehreren der Ansprüche 1 bis 5, in dem das System durch die Zugabe von Blutgerinnungsfaktor XII, Plasmapräkallikrein und einem Kininogen, das ein hohes Molekulargewicht aufweist, aufbereitet wird.

8. Verfahren gemäß einem der mehreren der Ansprüche 1 bis 4, 6 und 7, in dem die hergestellte physiologisch aktive Substanz durch Zugabe eines Substrates bestimmt wird.

9. Verfahren gemäß Ansprüche 8, in dem das Substrat ein synthetisches Substrat ist.

10. Verwendung eines in einem aufbereiteten Plasma vorliegenden Kallikrein-Kinin-Systems, das
i) den Blutgerinnungsfaktor XII,
ii) Plasmapräkallikrein,
iii) gegebenenfalls Kininogen, das ein hohes Molekulargewicht aufweist, umfasst, und
iv) keine anderen Inhibitoren oder Promotoren des enthält,
zur Messung der Aktivität einer Testsubstanz.

11. Verwendung gemäß Anspruch 10, worin die Testsubstanz ein Arzneimittel ist.

## Revendications

1. Méthode de mesure de l'activité d'une substance d'essai, caractérisée par les étapes suivantes :
i) lancement d'une série de réactions enzymatiques dans un système kallikréine-kinine de plasma reconstitué, en présence de la substance d'essai, dans laquelle l'activation du facteur XII de coagulation du sang est la réaction initiale,
ii) arrêt de la réaction, et
iii) détermination quantitative de la substance physiologiquement active produite dans la réaction ci-dessus.

2. Méthode selon la revendication 1, dans laquelle la production de la substance physiologiquement active est arrêtée avant que cette production ne devienne saturée.

3. Méthode selon les revendications 1 ou 2, dans laquelle la substance physiologiquement active est le facteur XII de coagulation du sang sous une forme active.

4. Méthode selon les revendications 1 ou 2, dans laquelle la substance physiologiquement active est la kallikréine de plasma.

5. Méthode selon les revendications 1 ou 2, dans laquelle la substance physiologiquement active est la bradykinine.

6. Méthode selon l'une quelconque des revendications 1 à 4, dans laquelle le système est reconstitué par l'addition du facteur XII de coagulation du sang et de la prékallikréine de plasma.

7. Méthode selon l'une des revendications 1 à 5, dans laquelle le système est reconstitué par l'addition du facteur XII de coagulation du sang, de la prékallikréine de plasma et d'un kininogène de masse moléculaire élevée.

8. Méthode selon l'une quelconque des revendications 1 à 4, 6 et 7, dans laquelle la substance physiologiquement active produite est déterminée par l'addition d'un substrat.

9. Méthode selon la revendication 8, dans laquelle le substrat est un substrat synthétique.

10. Utilisation d'un système kallikréine-kinine de plasma reconstitué comprenant :
i) le facteur XII de coagulation du sang
ii) de la prékallikréine de plasma,
iii) comprenant facultativement un kininogène de masse moléculaire élevée, et
iv) ne contenant pas d'autres inhibiteurs ou promoteurs du système réactionnel kallikréine-kinine de plasma
en vue de la mesure de l'activité d'une substance d'essai.

11. Utilisation selon la revendication 10, dans laquelle la substance d'essai est un médicament.
